# EUROPEAN PATENT APPLICATION

(11) **EP 3 872 188 A1**
(43) Date of publication of application: **01.09.2021**
(21) Application number: 19875234.7
(22) Date of filing: 17.10.2019
(51) Int. Cl.: C12Q 1/6825, C12M 1/34

(54) **METHOD FOR TREATING SOLUTION INCLUDING NUCLEIC ACID, AND DEVICE FOR TREATING SOLUTION INCLUDING NUCLEIC ACID**

(30) Priority: 25.10.2018 JP 2018200570
(71) Applicant: YOKOWO CO., LTD., Kita-ku Tokyo 114-8515 (JP)
(72) Inventor: TAKASE, Shintaro, Tokyo 114-8515 (JP); IKEDA, Hisafumi, Tokyo 173-8602 (JP); MIYAGAWA, Kouta, Tokyo 114-8515 (JP); USAMI, Eiji, Tokyo 114-8515 (JP)
(74) Representative: Sibley, Lara Ann
(86) International application number: PCT/JP2019/040922
(87) International publication number: WO 2020/085199

(57) **Abstract**

A first base material (100) is prepared. The first base material (100) has a first surface (102). The first surface (102) includes a well (102a). The well (102a) is defined by an electrode (120) and a resist (130). Next, a solution (L) is arranged on the first surface (102) of the first base material (100). Next, a second base material (200) is prepared. The second base material (200) has a second surface (202). The second surface (202) of the second base material (200) is hydrophobic. Next, the first base material (100) and the second base material (200) are pressed together so that the first surface (102) and the second surface (202) face each other.

## Description

### FIELD

The preset invention relates to a method for treating a solution including nucleic acid and a device for treating a solution including nucleic acid.

### BACKGROUND

Various devices for treating solutions including nucleic acid have been proposed. These devices can electrochemically analyze the nucleic acid contained in the solution. In electrochemical analysis of nucleic acid, the nucleic acid hybridizes with a probe immobilized on an electrode in the device. In this case, the degree of hybridization of the nucleic acid can be quantitatively analyzed based on a comparison of electrochemical analyses before hybridization and after hybridization (e.g., cyclic voltammetry (CV) measurement).

Non-Patent Literature 1 describes an example of a device for treating a solution including nucleic acid. This device has a well defined by an electrode and a resist. Such a device can be impregnated with the solution including nucleic acid. In this case, the solution is arranged from the interior of the well to the exterior of the well.

### [CITATION LIST]

### [NON PATENT LITERATURE]

[NPL 1] Hiroshi AOKI, Akiko KITAJIMA, and Hiroaki TAO "Electrochemical Sensor Array Chips for Multiple Gene Detection", Sensors and Materials, Vol. 22, No. 7 (2010), pp. 327-336

### SUMMARY

### [TECHNICAL PROBLEM]

As described above, wells are sometimes used in the treatment of solutions including nucleic acid. The present inventors have examined the electrochemical analysis of a small target copy number of nucleic acid using a well. For example, when a solution is arranged from the interior of a well to the exterior of the well, as described above, the target copy number of the nucleic acid may be large.

In particular, it has been difficult to detect microRNA (miRNA) specific to cancer patients by the conventional method described above. For example, the amount of solution arranged from the interior of the well to the exterior of the well is at least approximately 10 µL in the conventional method. In this case, when the minimum nucleic acid concentration required to obtain complementary nucleic acid hybridization is 4 nM, the target copy number of the nucleic acid is approximately 400 amol. Since the total amount of miRNA obtained from 10 mL of serum of a cancer patient is approximately 1 amol or less, as will be described later, it is difficult to detect miRNA specific to a cancer patient by the conventional method.

Electrochemical analysis of a small target copy number of nucleic acid is an example of an object of the present invention. Other objects of the present invention will be clarified from the descriptions of the present specification.

### [SOLUTION TO PROBLEM]

An aspect of the present invention provides:
a method for treating a solution including nucleic acid, comprising the steps of:
arranging the solution on a first surface of a first base material having the first surface, which has a well defined by an electrode and a resist; and
after arranging the solution on the first surface of the first base material, pressing the first base material and a second base material, which has a hydrophobic second surface, together so that the second surface of the second base material and the first surface of the first base material face each other.

Another aspect of the present invention provides:
a device for treating a solution including nucleic acid, comprising:
a first base material having a first surface having a well defined by an electrode and a resist;
a second base material having a hydrophobic second surface; and
a member for pressing the first base material and the second base material together so that the first surface and the second surface face each other.

Yet another aspect of the present invention provides:
a device for treating a solution including nucleic acid, comprising:
a first base material having a first surface having a well defined by an electrode and a resist; and
a second base material having a hydrophobic second surface, wherein
the first base material and the second base material are joined to each other so that the first surface and the second surface face each other.

### [ADVANTAGEOUS EFFECTS OF INVENTION]

According to the aspects of the present invention described above, a small target copy number of nucleic acid can be electrochemically analyzed.

### BRIEF DESCRPITION OF DRAWINGS

FIG. 1 is a view detailing an example of a solution treatment method according to an embodiment.
FIG. 2 is a view detailing an example of a solution treatment method according to an embodiment.
FIG. 3 is a view detailing an example of a solution treatment method according to an embodiment.
FIG. 4 is a view detailing an example of a solution treatment method according to an embodiment.
FIG. 5 is an example of a plan view of a first base material.
FIG. 6 is a view detailing a first example of a solution treatment device according to an embodiment.
FIG. 7 is a view detailing a second example of a solution treatment device according to an embodiment.
FIG. 8 is a view detailing a potential difference ΔE calculated in the Examples.
FIG. 9 is a view showing calculation results of the potential difference ΔE in the Examples.
FIG. 10 is a view showing calculation results of the potential difference ΔE in the Examples.
FIG. 11 is a view showing calculation results of the potential difference ΔE in the Examples.

### DESCRIPTION OF EMBODIMENTS

The embodiments of the present invention will be described below using the drawings. In the drawings, identical constituent elements are assigned the same reference sign, and descriptions have been omitted as appropriate.

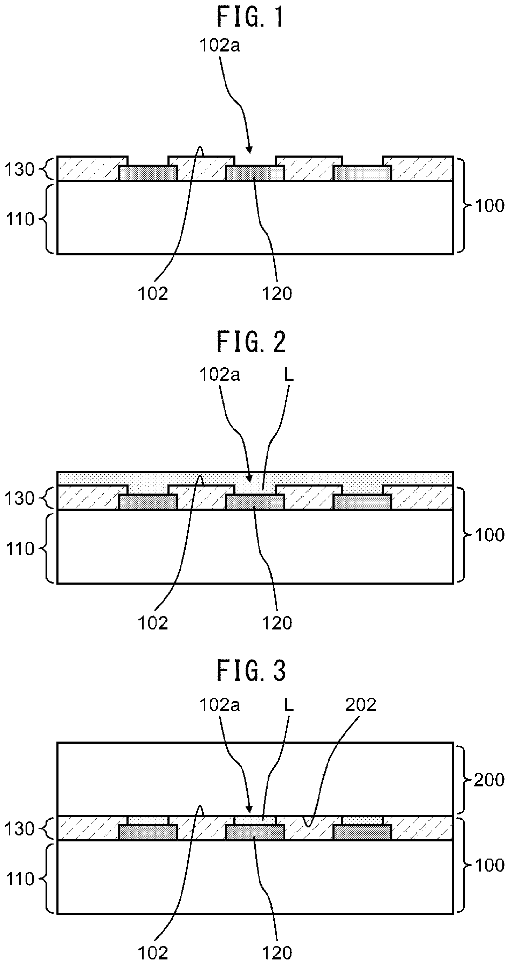

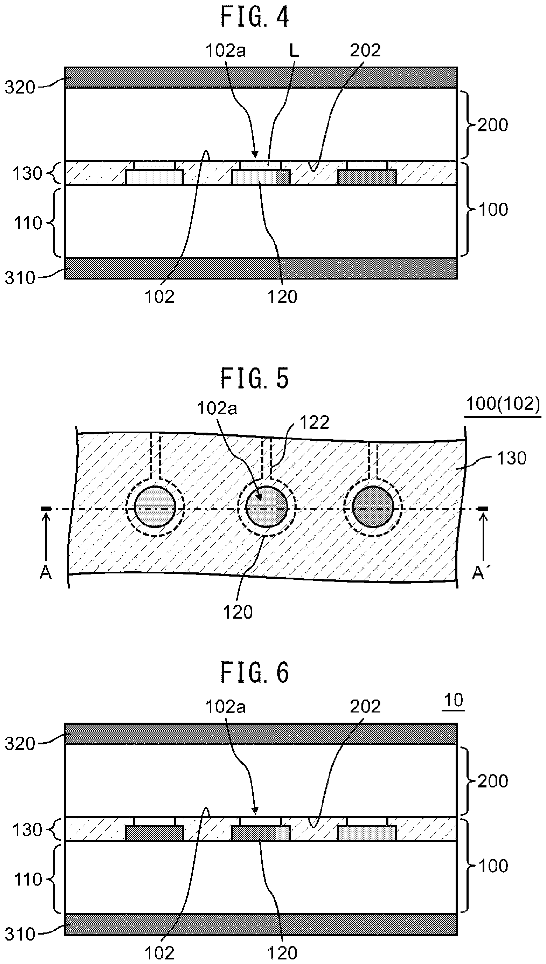

FIGS. 1 to 4 are views detailing examples of a solution treatment method according to an embodiment. FIG. 5 is an example of a plan view of a first base material 100. FIGS. 1 to 4 show cross sections of cross-sections A-A' of FIG. 5.

Using FIGS. 1 to 4, a summary of the solution treatment method according to the embodiment will be described. First, a first base material 100 is prepared as shown in FIG. 1. The first base material 100 has a first surface 102. The first surface 102 includes a well 102a. The well 102a is defined by an electrode 120 and a resist 130. Next, a solution L is arranged on the first surface 102 of the first base material 100 as shown in FIG. 2. The solution L includes nucleic acid. Next, a second base material 200 is prepared as shown in FIG. 3. The second base material 200 has a second surface 202. The second surface 202 of the second base material 200 is hydrophobic. Next, as shown in FIG. 4, the first base material 100 and the second base material 200 are pressed together so that the first surface 102 and the second surface 202 face each other. In the example shown in FIG. 4, the first base material 100 and the second base material 200 are pressed together using a first member 310 and a second member 320. In another example, the first base material 100 and the second base material may be pressed together by a method different from the method shown in FIG. 4 (for example, the second base material 200 is pressed toward the first base material 100 in a state in which the first base material 100 is arranged on a fixed base).

According to the solution treatment method according to the embodiment, a small target copy number of nucleic acid can be electrochemically analyzed. Specifically, in the solution treatment method of the embodiment, as shown in FIG. 4, the solution L contacting each electrode 120 can remain only in the well 102a. In other words, the solution L remains in the cavity defined by the electrode 120, the resist 130, and the second surface 202 of the second base material 200 (the solution L contacts only the electrode 120, the resist 130, and the second surface 202). Specifically, the second surface 202 of the second base material 200 is hydrophobic, whereby leakage of the solution L inside the well 102a to the outside due to capillary action can be suppressed. Thus, according to the solution treatment method according to the embodiment, the target copy number of the nucleic acid in the solution L in contact with each electrode 120 can be limited in accordance with the volume of the well 102a. Therefore, a small target copy number of nucleic acid can be electrochemically analyzed.

In the embodiments and Examples, the solution including nucleic acid includes a hybridization solution and nucleic acid (e.g., RNA (e.g., miRNA) or DNA).

As shown in FIGS. 3 and 4, the first surface 102 of the first base material 100 includes a surface of the resist 130, in which the surface faces the second surface 202 of the second base material 200. Thus, when the first base material 100 and the second base material 200 are pressed together, the surface of the resist 130 facing the second surface 202 and the second surface 202 of the second base material 200 contact each other.

The details of an example of a plan layout of the first base material 100 will be described using FIG. 5. The plan layout shown in FIG. 5 is merely an example of a plan layout of the first base material 100. The first base material 100 may have a plan layout different from the plan layout shown in FIG. 5.

The first base material 100 comprises a plurality of electrodes 120, a plurality of wiring 122, and the resist 130.

The resist 130 has a plurality of openings. In the example shown in FIG. 5, each opening of the resist 130 has a circular shape. In another example, each opening of the resist 130 may have a shape different from the circular shape.

A portion of each electrode 120 is exposed from respective opening of the resist 130. Thus, each well 102a is defined by each electrode 120 and the resist 130. In the examples shown in FIGS. 1 to 5, the wells 102a have a cylindrical shape.

One end of each wiring 122 is connected to respective electrode 120. The other end of each wiring 122 may be connected to a terminal (not illustrated) for acquiring electronic signals of the electrode 120.

The details of the solution treatment method according to the present embodiment will be described using FIGS. 1 to 4.

First, the first base material 100 is prepared as shown in FIG. 1. The first base material 100 has a substrate 110, a plurality of electrodes 120, and the resist 130.

The substrate 110 may be a glass substrate, may be a semiconductor substrate (e.g., a silicon substrate), or may be a resin substrate. In another example, the first base material 100 may have, in place of the substrate 110, a member (e.g., a member having a shape different from plate-like) having a surface on which the electrodes 120 and the resist 130 can be formed.

The plurality of electrodes 120 are on the substrate 110. The electrodes 120 are made of a conductive material, for example, a metal. The electrodes 120 are capable of functioning as working electrodes.

The resist has a plurality of openings. Each opening of the resist 130 exposes a portion of respective electrode 120. The resist 130 is made of an insulating material, for example, a resin.

The electrode 120 has a surface which is exposed from the resist 130. In an example, the exposed surface of the electrode 120 may be hydrophilic. In this case, in the arrangement of the solution L of FIG. 2, the solution L can easily spread on the exposed surface of the electrode 120. The hydrophilicity of the exposed surface of the electrode 120 may be imparted by selectively hydrophilizing the exposed surface of the electrode 120 by, for example, using a mask which covers the resist 130 and has an opening which overlaps the electrode 120.

The surface of the resist 130 may be less hydrophilic than the exposed surface of the electrode 120, e.g., may be hydrophobic.

The first base material 100 has a first surface 102, and the first surface 102 has a plurality of wells 102a. Each well 102a is defined by an electrode 120 and the resist 130. Specifically, the electrodes 120 form the bottom surfaces of the wells 102a, and the resist 130 forms the inside surfaces of the wells 102a.

In an example, the volume of each well 102a may be 1 nL or less. In this case, in the confinement of the solution L in FIG. 4, the amount of solution L in each well 102a can be reduced.

In the example shown in FIG. 1, the first base material 100 has a plurality of electrodes 120 (specifically, a plurality of wells 102a). In another example, the first base material 100 may have only one electrode 120 (specifically, only one well 102a).

Next, as shown in FIG. 2, the solution L is arranged on the first surface 102 of the first base material 100. The solution L includes nucleic acid.

In the example shown in FIG. 2, the solution L is arranged from the interior of the well 102a to the exterior of the well 102a (in other words, a portion of the solution L is arranged outside the well 102a, e.g., arranged so as to cover the first surface 102). In this example, by pressing the first base material 100 and the second base material 200 of FIGS. 3 and 4 together, the solution L outside the well 102a can be discharged outside of the first base material 100 and the second base material 200. In the example shown in FIG. 2, the solution L arranged outside the well 102a may be partially separated.

In another example, none of the parts of the solution L may be arranged inside the well 102a (in other words, the entirety of the solution L is arranged outside the well 102a). In this example, the solution L outside the well 102a can be caused to enter the well 102a by pressing the first base material 100 and the second base material 200 together in FIGS. 3 and 4.

In yet another example, the solution L may be provided to each of the plurality of wells 102a. The solution L can be applied to each well 102a by, for example, dropping. In this example, the solution L in each well 102a can be dropped in an amount substantially equal to or greater than the volume of the well 102a. When the volume of the solution L in each well 102a is greater than the volume of the well 102a, the surface of the solution L may protrude at a position higher than the first surface 102 (upper surface of the resist 130) of the first base material 100. In this example, as shown in FIGS. 3 and 4, when the well 102a is covered with a hydrophobic surface, i.e., the second surface 202 of the second base material 200, drying of the fluid L can be suppressed. In addition, the volume of the solution L confined in each well 102a can be made constant, which allows the degrees of hybridization between wells 102a to be compared. Further, when the hydrophobic surface (the second surface 202 of the second base material 200) covers the well 102a, leakage of the solution L in the well 102a due to capillary action can be suppressed as compared with the case in which the hydrophilic surface covers the well 102a.

The second base material 200 is then prepared as shown in FIG. 3.

In the example shown in FIG. 3, the second base material 200 is a substrate and has a plate-like shape. In another example, the second substrate 200 may have a different shape from a plate-like shape.

The second surface 202 of the second base material 200 is hydrophobic. Specifically, the second surface 202 of the second base material 200 has a water contact angle of 90° or more. If the second surface 202 of the second base material 200 is hydrophilic, the solution L in the well 102a may leak to the outside of the first base material 100 and the second base material 200 by capillary action through the gap between the first base material 100 and the second base material 200. Conversely, when the second surface 202 of the second base material 200 is hydrophobic, leakage of the solution L in the well 102a to the outside of the first base material 100 and the second base material 200 can be suppressed, and the solution L can be retained in the well 102a with high reliability.

In one example, the second surface 202 of the second base material 200 is made of a hydrophobic material, e.g., polytetrafluoroethylene. In another example, the second surface 202 of the second base material 200 may be hydrophobized. The entire second base material 200 may not be hydrophobic or the entire second base material 200 may be hydrophobic.

Next, the first base material 100 and the second base material 200 are pressed against each other as shown in FIG. 4 so that the first surface 102 and the second surface 202 face each other.

In the example shown in FIG. 4, the first base material 100 is pressed toward the second base material 200 by the first member 310, and the second base material 200 is pressed toward the first base material 100 by the second member 320. Specifically, the first member 310 and the second member 320 are members for pressing the first base material 100 and the second base material 200 together. In one example, the first member 310 and the second member 320 may be clips.

By pressing the first member 310 and the second member 320 together, the solution L can be retained in the well 102a with high reliability. In one example, when the solution L is arranged from the interior of the well 102a to the exterior of the well 102a before the first base material 100 and the second base material 200 are pressed together, the solution L outside the well 102a can be discharged to the outside of the first base material 100 and the second base material 200. In another example, if the solution L is not arranged inside the well 102a before the first base material 100 and the second base material 200 are pressed together, the solution L outside the well 102a can enter the well 102a.

The solution treatment method shown in FIGS. 1 to 4 can be applied to various examples of the treatment of a solution containing nucleic acid, and can be applied to, for example, the hybridization of nucleic acid.

An example in which the solution treatment method shown in FIGS. 1 to 4 is applied to the hybridization of nucleic acid will be described below.

In this example, while the first base material 100 and the second base material 200 are pressed against each other (e.g., FIG. 4), the nucleic acid in the solution L can be hybridized to a probe immobilized on the electrode 120. In this example, a small target copy number of the nucleic acid can be hybridized.

Further, prior to hybridizing the nucleic acid with the probe, specifically, prior to arrangement of the solution L on the first surface 102 of the first base material 100 (FIG. 2) and after immobilization of the probe and a thiol (thiols are substances which do not bind to the target nucleic acid and which physically support the structure of the probe, e.g., 6-hydroxy-1-hexanethiol (HHT)) on the electrode 120, electrochemical analysis may be performed using the electrode 120. In one example, a voltammogram may be measured by CV from the electrode 120.

Further, after hybridizing the nucleic acid with the probe, specifically, after removing the second base material 200 from the first base material 100 and washing the first base material 100, electrochemical analysis may be performed using the electrode 120. In one example, a voltammogram may be measured by CV from the electrode 120. In this example, a small target copy number of nucleic acid can be electrochemically analyzed.

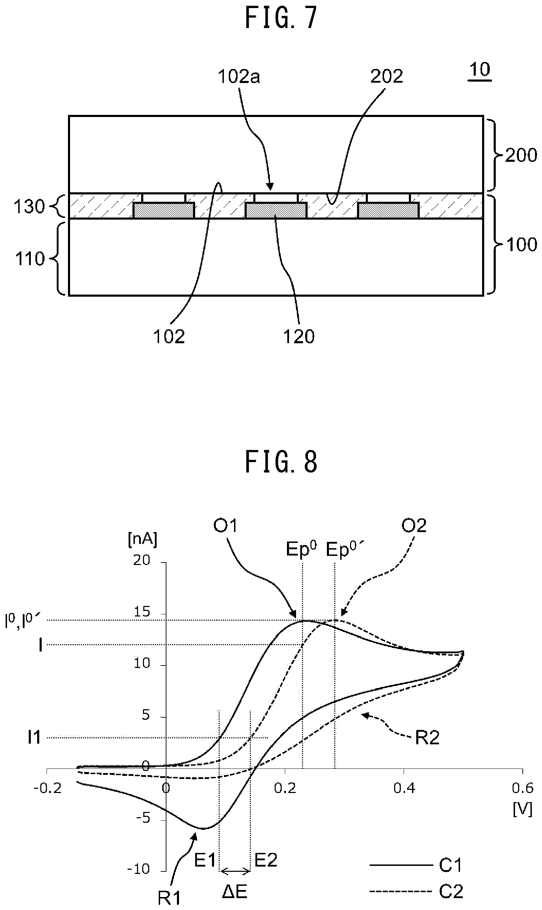

In the example described above, the degree of hybridization of the nucleic acid can be determined based on a compassion between the electrochemical analysis before hybridization (e.g., the voltammogram measured by CV) and the electrochemical analysis after hybridization (e.g., the voltammogram measured by CV) (e.g., a potential difference ΔE, which is described later using FIG. 8).

The solution treatment method according to the present embodiment is applicable not only to the CV described above but also to electrochemical analysis other than CV (e.g., SWV (Square Wave Voltammetry)).

In one example, the nucleic acid may be a microRNA (miRNA). miRNA may be taken from blood. Generally, it is difficult to obtain a sample containing a large amount of miRNA from blood. According to the solution treatment method shown in FIGS. 1 to 4, a small target copy number of miRNA can be electrochemically analyzed.

FIG. 6 is a view detailing a first example of a solution treatment device 10 according to the embodiment.

The solution treatment device 10 includes a first base material 100, a second base material 200, a first member 310, and a second member 320. The first member 310 and the second member 320 are members for pressing the first base material 100 and the second base material 200 together so that the first surface 102 and the second surface 202 face each other. When the first member 310 and the second member 320 are not provided, the first base material 100 and the second base material 200 are spaced apart from each other. Thus, using the solution treatment method shown in FIGS. 1 to 4, the solution treatment device 10 can be used to electrochemically analyze a small target copy number of nucleic acid.

FIG. 7 is a view detailing a second example of a solution treatment device 10 according to the embodiment.

The solution treatment device 10 includes a first base material 100 and a second base material 200. The first base material 100 and the second base material 200 are joined together so that the first surface 102 and the second surface 202 face each other. The first base material 100 and the second base material 200 may be bonded to each other, for example, via an adhesive layer. In the example shown in FIG. 7, the solution can be introduced into the well 102a via an introduction path (not shown in FIG. 7) leading to the well 102a. In the example shown in FIG. 7, a small target copy number of nucleic acid can be electrochemically analyzed.

### EXAMPLES

FIG. 8 is a view detailing the potential difference ΔE calculated in the embodiment.

In the example, the potential difference ΔE is calculated by the following process.

First, a first base material 100 is prepared as shown in FIG. 1, and a probe having a sequence complementary to the target miRNAs and a thiol are immobilized on the electrode 120. The electrode 120 is then used to measure voltammogram C1 (FIG. 8) by CV measurement.

The solution containing the target miRNA (which solution contains the target miRNA and the hybridization solution) is then dropped onto the first surface 102 of the first base material 100, as shown in FIG. 2.

The clips (first member 310 and second member 320) then push the first base material 100 and second base material 200 together, as shown in FIGS. 3 and 4. In this manner, the excess solution L can be extruded to the outside of the first base material 100 and the second base material 200 while the solution is retained in the well 102a.

The miRNA is then hybridized by heating the first base material 100 and the second base material 200 while the first base material 100 and the second base material 200 are pressed together by the clips (first member 310 and second member 320).

The clips (first member 310 and second member 320) are then removed from the first base material 100 and the second base material 200, and the second base material 200 is removed from the first base material 100. The first surface 102 of the first base material 100 is then cleaned.

The electrode 120 is then used to measure voltammogram C2 (FIG. 8) by CV measurement.

As shown in FIG. 8, voltammogram C1 includes a first oxidation wave O1 and a first reduction wave R1, and voltammogram C2 includes a second oxidation wave 02 and a second reduction wave R2.

The first oxidation wave O1 has a peak current value I⁰ at potential Ep⁰. The first oxidation wave O1 has a current value I1 at potential E1 (E1 < Ep⁰).

The second oxidation wave 02 has a peak current value I^{0'} at potential Ep^{0'}. The second oxidation wave 02 has a current value I at potential Ep⁰. The second oxidation wave 02 has current value I1 at the potential E2 (E2 < Ep^{0'}).

The potential difference ΔE is the difference between the potential E1 of the first oxidation wave O1 and the potential E2 of the second oxidation wave 02.

The reason why the potential difference ΔE occurs is as follows. The potential of the electrode 120 (the working electrode) may be reduced by the negative total charge amount ΔQ generated by the hybridized target nucleic acid. In the measurement of the oxidation waves, an electric double layer of capacitance C can be formed on the working electrode. The fall in the potential of the working electrode can be estimated as ΔQ/C. Thus, the oxidation wave after hybridization (in the example shown in FIG. 8, the second oxidation wave 02) may shift from the oxidation wave before hybridization (in the example shown in FIG. 8, the first oxidation wave O1) towards a high potential by ΔQ/C. The potential difference ΔE can be estimated as the amount of shift from the oxidation wave before hybridization (in the example shown in FIG. 8, the first oxidation wave O1) to the oxidation wave after hybridization (in the example shown in FIG. 8, the second oxidation wave 02), and is approximately equivalent to ΔQ/C. Thus, the potential difference ΔE can be an indicator for quantitatively analyzing the degree of hybridization of nucleic acid.

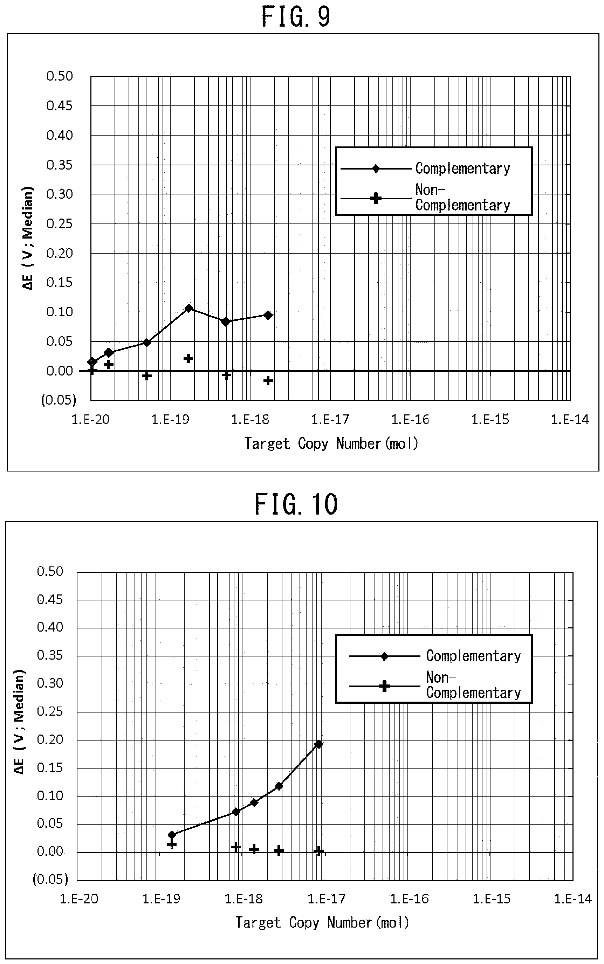

FIGS. 9 to 11 are views showing the calculation results of the potential difference ΔE in the embodiment.

In FIGS. 9 to 11, miRNA was hybridized to the probe while the first base material 100 and the second base material 200 were pressed together (FIG. 4) by the clips (first member 310 and second member 320).

In FIG. 9, the volume of the well 102a was 2.1 pL (the diameter of the well 102a: 30 µm, the depth of the well 102a: 3 µm). For each of miRNA complementary to the probe and miRNA non-complementary to the probe, samples containing the following target copy numbers of nucleic acid were measured. Each plot of the complementary miRNA in FIG. 9 shows the median of the 75 potential differences ΔE calculated using the respective 75 measurement systems (75 electrodes 120). Similarly, each plot of the non-complementary miRNA in FIG. 9 shows the median of the 75 potential differences ΔE calculated using the respective 75 measurement systems (75 electrodes 120).
11 zmol (5 nM × 2.1 pL)
17 zmol (8 nM × 2.1 pL)
51 zmol (24 nM × 2.1 pL)
168 zmol (80 nM × 2.1 pL)
509 zmol (240 nM × 2.1 pL)
1.7 amol (800 nM × 2.1 pL)

In FIG. 10, the volume of the well 102a was 35 pL, (the diameter of the well 102a: 67 µm, the depth of the well 102a: 10 µm). For each of miRNA complementary to the probe and miRNA non-complementary to the probe, samples containing the following target copy numbers of nucleic acid were measured. Each plot of the complementary miRNA in FIG. 10 shows the median of the 75 potential differences ΔE calculated using the respective 75 measurement systems (75 electrodes 120). Similarly, each plot of the non-complementary miRNA in FIG. 10 shows the median of the 75 potential differences ΔE calculated using the respective 75 measurement systems (75 electrodes 120).
141 zmol (4 nM × 35 pL)
0.84 amol (24 nM × 35 pL)
1.4 amol (40 nM × 35 pL)
2.8 amol (80 nM × 35 pL)
8.4 amol (240 nM × 35 pL)

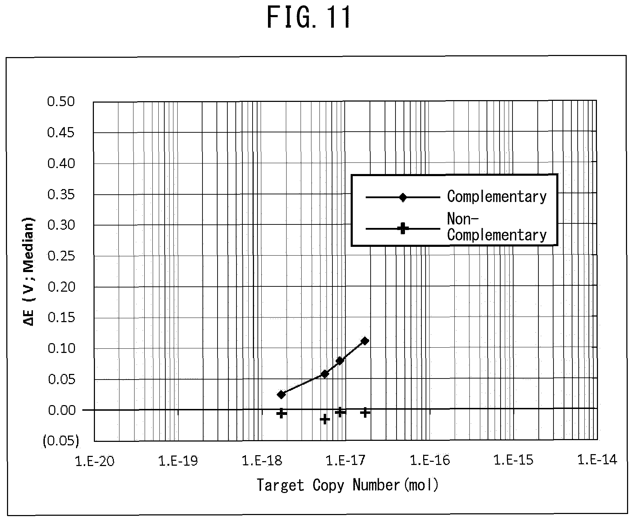

In FIG. 11, the volume of the well 102a was 71 pL, (the diameter of the well 102a: 95 µm, the depth of the well 102a: 10 µm). For each of miRNA complementary to the probe and miRNA non-complementary to the probe, samples containing the following target copy numbers of nucleic acid were measured. Each plot of the complementary miRNA in FIG. 11 shows the median of the 75 potential differences ΔE calculated using the respective 75 measurement systems (75 electrodes 120). Similarly, each plot of the non-complementary miRNA in FIG. 11 shows the median of the 75 potential differences ΔE calculated using the respective 75 measurement systems (75 electrodes 120).
1.7 amol (24 nM × 71 pL)
5.7 amol (80 nM × 71 pL)
8.5 amol (120 nM × 71 pL)
17 amol (240 nM × 71 pL)

From the results shown in FIG. 9, it can be estimated that the detection limit of the target copy number in the well 102a of FIG. 9 is approximately 17 zmol.

From the results shown in FIG. 10, it can be estimated that the detection limit of the target copy number in the well 102a of FIG. 10 is approximately 141 zmol.

From the results shown in FIG. 11, it can be estimated that the detection limit of the target copy number in the well 102a in FIG. 11 is approximately 1.7 amol.

The results shown in FIGS. 9 to 11 suggest that electrochemical analysis of a small target copy number of miRNA can be performed by pressing the first base material 100 and the second base material 200 together.

Furthermore, in any of FIGS. 9 to 11, the limit of detection has nearly reached 1 amol. Thus, the methods illustrated in FIGS. 9 to 11 can be utilized to detect miRNA in blood. The total amount of miRNA (one type) in cancer patients present in 10 mL of serum (20 mL of blood) is approximately 1 amol or less. Therefore, a detection limit of approximately 1 amol or less is required for the detection of miRNA in blood. As noted above, in any of the methods illustrated in FIGS. 9 to 11, the limit of detection has almost reached 1 amol.

As is clear from the descriptions herein, the object of the present invention is not limited to the detection of miRNA unique to cancer patients. Each aspect of the present invention is also applicable to the detection of nucleic acid other than miRNA specific to cancer patients.

While embodiments of the present invention have been described above with reference to the accompanying drawings, these embodiments are illustrative of the present invention, and various configurations other than those described above may be used.

The present application claims priority based on Japanese Patent Application No. 2018-200570, filed Oct. 25, 2018, the disclosure of which is incorporated herein in its entirety.

### REFERENCE SIGNS LIST

- 10: solution treatment device
- 100: first base material
- 102: first surface
- 102a: well
- 110: substrate
- 120: electrode
- 122: wiring
- 130: resist
- 200: second base material
- 202: second side
- 310: first member
- 320: second member

## Claims

1. A method for treating a solution including nucleic acid, comprising the steps of:
arranging the solution on a first surface of a first base material having the first surface, which has a well defined by an electrode and a resist, and
after arranging the solution on the first surface of the first base material, pressing the first base material and a second base material, which has a hydrophobic second surface, together so that the second surface of the second base material and the first surface of the first base material face each other.

2. The method for treating a solution according to claim 1, wherein the nucleic acid contained in the solution is hybridized with a probe immobilized on the electrode in a state in which the first base material and the second base material are pressed together.

3. The method for treating a solution according to claim 2, further comprising executing electrochemical analysis using the electrode prior to hybridization of the nucleic acid with the probe, and
executing electrochemical analysis using the electrode after hybridization of the nucleic acid with the probe.

4. The method for treating a solution according to claim 2 or 3, wherein the nucleic acid is RNA or DNA.

5. The method for treating a solution according to claim 4, wherein the nucleic acid is miRNA.

6. The method for treating a solution according to any one of claims 1 to 5, wherein the solution is arranged on the first surface of the first base material so that at least part of the solution is arranged outside the well.

7. The method for treating a solution according to any one of claims 1 to 6, wherein the first base material and the second base material are pressed together so that the solution in the well contacts the electrode, the resist, and the second surface of the second base material.

8. The method for treating a solution according to claim 7, wherein the volume of the well is 1 nL or less.

9. A device for treating a solution including nucleic acid, comprising:
a first base material having a first surface having a well defined by an electrode and a resist;
a second base material having a hydrophobic second surface; and
a member for pressing the first base material and the second base material together so that the first surface and the second surface face each other.

10. The device for treating a solution according to claim 9, wherein the member presses the first base material and the second base material together so that the resist and the second surface of the second base material contact each other.

11. A device for treating a solution including nucleic acid, comprising:
a first base material having a first surface having a well defined by an electrode and a resist; and
a second base material having a hydrophobic second surface, wherein
the first base material and the second base material are joined to each other so that the first surface and the second surface face each other.

12. The device for treating a solution according to claim 11, wherein the first base material and the second base material are joined to each other so that the resist and the second surface of the second base material contact each other.
